# EUROPEAN PATENT SPECIFICATION

(11) **EP 3 340 962 B1**
(45) Date of publication and mention of the grant of the patent: **15.09.2021**
(21) Application number: 16840997.7
(22) Date of filing: 29.07.2016
(51) Int. Cl.: A61K 8/27, A61K 8/35, A61K 9/14, A61Q 17/04

(54) **ENHANCED PHOTOSTABILITY, EXTENDED RANGE UVA FILTERING AND CAMOUFLAGING POTENTIAL OF AVOBENZONE-DEFECT RICH ZNO NANOCRYSTALS COMPLEX**
VERBESSERTE LICHTSTABILITÄT, UVA-FILTERUNG MIT ERWEITERTEM BEREICH UND TARNUNGSPOTENZIAL EINES AVOBENZON-DEFEKTREICHEN ZNO-NANOKRISTALLKOMPLEXES
COMPLEXE AVOBENZONE-NANOCRISTAUX DE ZNO RICHES EN DÉFAUTS PRÉSENTANT UNE PHOTOSTABILITÉ AMÉLIORÉE, UN POUVOIR ANTI-UVA À LARGE SPECTRE ET UN POTENTIEL DE MASQUAGE

(30) Priority: 29.08.2015 IN 3310MU2015
(43) Date of publication of application: 04.07.2018
(73) Proprietor: Indian Institute of Technology Bombay, Maharashtra 400 076 (IN)
(72) Inventor: ADERSH, Asok, Mumbai 400076 (IN); AJIT, R., Kulkarni, Mumbai Maharashtra 400076 (IN)
(74) Representative: EP&C
(86) International application number: PCT/IN2016/000198
(87) International publication number: WO 2017/037731

(56) References cited:
- WO-A1-2014/152096
- WO-A2-2011/063329
- US-A1- 2002 016 488
- US-A1- 2009 155 194
- US-A1- 2012 225 006
- US-A1- 2013 129 649
- US-A1- 2014 030 298
- FAMENGO A. ET AL.: 'Facile and Reproducible Synthesis of Nanostructured Colloidal ZnO Nanoparticles from Zinc Acetylacetonate: Effect of Experimental Parameters and Mechanistic Investigations' EUROPEAN JOURNAL OF INORGANIC CHEMISTRY vol. 2009, no. 33, November 2009, pages 5017 - 5028, XP055367733
- DJURISIC A. B. ET AL.: 'Defect emissions in ZnO nanostructures' NANOTECHNOLOGY vol. 18, no. 9, July 2007, pages 1 - 8, XP020119914

## Description

### FIELD OF INVENTION

This invention generally relates to the use of Avobenzone-defect rich ZnO nanocrystals complex as multifunctional active ingredient in sunscreen, cosmetic and dermatological compositions, and more particularly to photostable sunscreen compositions and their use in improving the UV protection, aesthetic and natural appearance of the biological surface.

### BACKGROUND OF INVENTION

Exposure to ultraviolet (UV) radiation is the main causal factor for epidermal skin cells to become cancerous cells. Therefore, the functionality associated with sun protection compositions is to protect human skin and other tissue from the deleterious effects of these UV rays. The UV region, an electromagnetic radiation which covers the wavelength range of 400 nm to 100 nm, is further divided into three bands namely UVA (315-400 nm), UVB (280-315 nm), and UVC (100-280 nm). UVA is further divided into two wavelength ranges namely UVA1 (340-400 nm) and UVA2 (320-340 nm).

Existing sunscreen compositions typically contain a dispersion of inorganic compound or organic compound as UV filter (or) a combination of both inorganic and organic compound as UV filter. Among this inorganic UV filters are a preferred choice over the organic UV filter because the former possesses high photostablity when compared to the organic UV filter, which generally undergo photodegradation. Furthermore, the inorganic UV filters (ZnO/TiO₂) currently available are not effective enough for filtering the wavelength of UVA1 range, which is the main component of terrestrial UV radiation and reported to damage DNA and other biomolecules due to its ability to generate reactive oxygen species (ROS). Hence the FDA recommends the usage of broadband sunscreens whose application enables protection from UVA1 wavelength for preventing the epidermal skin cells from becoming cancerous.

There also exist mixtures of inorganic and organic UV filters forming an active ingredient of the sunscreen formulation which can filter the entire spectrum of UV rays. Now, photodegradation of organic UV filters in the presence of physical blockers like ZnO/TiO₂ deter the sunscreens' protection capability and proves to be a huge setback for the formulation in totality. A dibenzoylmethane derivative called Avobenzone (INCI name Butyl Methoxydibenzoylmethane and trade names Parsol® 1789, Escalol® 517, Eusolex® 9020) is a good example for the mentioned photostability issue.

The sunscreens with formulations comprising avobenzene as an active ingredient incorporate either surface modified inorganic filters or include additional stabilizers like diphenylacrylate, benzylidene camphor derivative, p-methoxycinnamate, octyl p-methoxycinnamate, octylmethoxycinnamate to address the issue of photostability. These inclusions only result in the increase of cost of production, affected cosmetic value and also leads to undesirable dermatological effects.

In order to effectively utilize the broadband UVA absorption of Avobenzene as opposed to other organic UV filters, which usually have a narrow spectrum of UV absorption, the problem of photostability in avobenzone requires to be mitigated. Reference is made also to the patent documents US 2013/0129649 and US 2009/0155194.

### SUMMARY OF INVENTION

This summary is provided to introduce concepts related to the usage of Avobenzone-defect rich ZnO nanocrystals complex (Av-DZnONCs) as an active ingredient for broadband UV protection as a topical application. The key concepts have been discussed below in detail.

In one aspect of the invention, a photostable Av-DZnONCs complex for broadband UV screening application with enhanced photoprotection is disclosed. This complex is an active ingredient of topical composition with cosmetic and pharmaceutical relevance.

In another aspect of the invention, is to introduce the application potential of Av-DZnONCscomplex/ defect rich ZnO quantum dots (DQDs) in improving the complexion of skin/correcting skin imperfection by brightening effect induced by the defect emission.

In yet another aspect of the invention, defect luminescence of Av-DZnONCs complex/D-QDs is employed in the method of predicting the sun protection factor when applied to a biological surface.

### BRIEF DESCRIPTION OF DRAWINGS

The figures listed below have been explained in detail in this section.
Figure 1a is representative of the TEM images of C-ZnO NPs, the bottom right inset shows the HR-TEM of ZnO NPs. Figure 1b is representative of the TEM images of D-QDs, the bottom right inset shows the HR-TEM of D-QDs (the circle show the exact location of defects, arrow is for eye guide) in accordance with an aspect of the present invention.
Figure 2 is representative of the Absorption spectrum of Avobenzone, D-QDs and defect rich ZnO quantum dots-avobenzone complex (D-QDs + Avobenzone) in accordance with an aspect of the present invention.
Figure 3 is representative of the Absorption spectrum of avobenzone with C-ZnO NPs, Avobenzone and C-ZnO NPs in accordance with an aspect of the present invention.
Figure 4 is representative of the Decay curve of the 419 nm emission of defect rich ZnO quantum dots-avobenzone complex (D-QDs + Avobenzone) and Avobenzone recorded with the time-correlated single photon counting (TCSPC; dots) under 395 nm laser excitation and bi-exponential fits to the data (solid lines) in accordance with an aspect of the present invention.
Figure 5 is representative of the FTIR spectrum showing broadened and shifted carbonyl peak ∼1618.38 cm⁻¹ for defect rich ZnO quantum dots-avobenzone complex (D-QDs + Avobenzone), which validate the formation of enol in accordance with an aspect of the present invention.
Figure 6 is representative of the Excitation spectra of defect rich ZnO quantum dots-avobenzone complex(D-QDs + Avobenzone), and D-QDs collected by fixing defect emission at 514 nm in accordance with an aspect of the present invention.
Figure 7 is representative of the Emission spectra of defect rich ZnO quantum dots-avobenzone complex (D-QDs + Avobenzone), and D-QDs collected under 350 nm excitation in accordance with an aspect of the present invention.
Figure 8 is representative of the absorbance spectrum showing the stability of defect rich ZnO quantum dots-avobenzone complex (D-QDs + Avobenzone), 365 nm 4W UV lamp excitation monitored at different time period in accordance with an aspect of the present invention.
Figure 9 is representative of the absorption spectrum showing the UV degradation of Avobenzone in the presence of C-ZnO NPs monitored at different time periods in accordance with an aspect of the present invention.
Figure 10a is representative of the absorption and emission spectra of D-QDs. Figure 10b is representative of the schematics showing the UV attenuation in the presence of D-QDs/defect rich ZnO quantum dots-avobenzone complex as a sunscreen and penetration of UVC, UVB and UVA without sunscreen in accordance with an aspect of the present invention.
Figure 11a illustrates the absorption and emission spectra of C-ZnO NPs. Figure 11b schematics show the penetration of intense UVA emission from C-ZnO NPs till dermis and penetration of UVC, UVB and UVA without sunscreen in accordance with an aspect of the present invention.
Figure 12 illustrates the use of defect emission from D-QDs for camouflaging the normal skin colour in accordance with an aspect of the present invention.
Figure 13a illustrates a photographic image of C-ZnO NPs coated on a skin emission model (PMMA/D-QDs nanocomposite) under a 365 nm UV lamp excitation. Figure 13b illustrates the photographic image of D-QDs coated on a skin emission model under a 365 nm UV lamp excitation. Figure 13c illustrates the transparency of oleic acid capped D-QDs coated on a glass substrate and Figure 13d illustrates the yellowish-green luminescence under a 365 nm UV lamp excitation in accordance with an aspect of the present invention.
Figure 14 illustrates the schematic showing method of measuring the efficacy of sun protection using defect emission intensity as a measure of sun protection factor in accordance with an aspect of the present invention.
Figure 15 illustrates the schematic showing method of using defect rich NCs (D-QDs/Av-DZnONCs) for personalized camouflaging application in accordance with an aspect of the present invention.

### DETAILED DESCRIPTION

The present invention relates to the use of Avobenzone-defect rich ZnO nanocrystals complex (Av-DZnONCs) as an active ingredient in cosmetic/UVA, UVB and UVC protection applications. The particle size of Av-DZnONCs described in the present invention can be in the range 1-100 nm. The nanocomplexes of Av-DZnONCs with size less than 10 nm are called defect rich ZnO quantum dots-avobenzone complex (D-QDs + Avobenzohe).

The complex Av-DZnONCs shows improved photostability and extended UVA filtering as opposed to sunscreen formulations containing only ZnO or Avobenzone. The Av-DZnONCs complex has no emission in the UVA range, however the Av-DZnONCs complex emits in visible region of the radiation spectrum, by virtue of the defect energy levels thereby mimicking in vivo human skin emission. In lieu of the mentioned property of emitting in the visible range, the composition of the present disclosure is topically applied to the biological surface, including but not limited to skin, in an amount effective for improving the UV protection, aesthetic and natural appearance of the biological surface. The amount effective or effective amount for topical application maybe ranging from 0.001 mg/cm² to about 5 mg/cm² of active ingredient.

A one-pot approach which makes an advantageous use of surface defect rich ZnO quantum dots (D-QDs), for stabilization of avobenzone has been utilized in the present invention thereby averting the issues associated with the erstwhile time consuming and costly pre-treatment steps adopted for stabilizing avobenzone.

### Example 1:

In a typical synthesis, 3 mL of Zn(OAc)₂ (0.05 M) in ethanol solution was complexed with 0.5 mL of Avobenzone (0.01 M) in ethanol solution. This complex was transferred into a 10 mL quartz tube and to this 3 mL of NaOH (0.05 M) was added at 0 °C to prevent the reaction during mixing. Then the quartz tube containing the reaction mixture was sealed by using a polymer cap. The hydrolyzing reaction was initiated by microwave irradiation with magnetic stirring at 75°C by using a CEM Discover microwave reactor with an operating frequency of 2.45 GHz. This results in the formation of highly stable defect rich ZnO quantum dots-Avobenzone complex. Different value of SPF can be achieved by varying the concentration of Avobenzone during complex formation (i.e., 0.0001 M to 1 M).

### Example 2:

In a typical synthesis 7 mL of defect rich ZnO quantum dots (0.0 25 M) was transferred into a quartz tube and to this 580 µL of Avobenzone (0.01M) was added. The entire mixture will readily form highly stable defect rich ZnO quantum dots-Avobenzone complex at room temperature. Different value of SPF can be achieved by varying the concentration of Avobenzone during complex formation (i.e., 0.0001 M to 1 M).

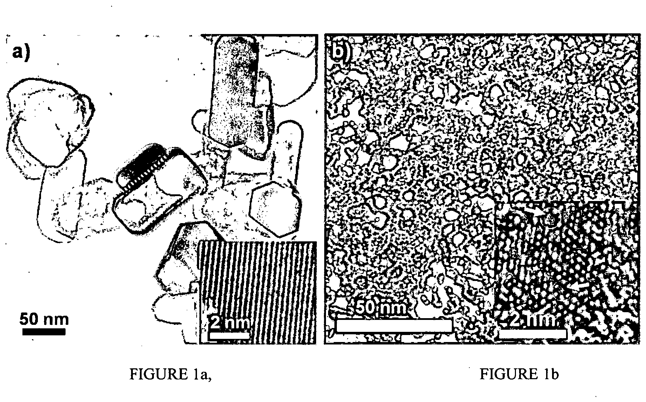

Now, Figure 1 is a comparative study of crystalline ZnO nanoparticle (C-ZnO NPs) along with D-QDs to bring out the effectiveness of D-QDs in forming photostable multifunctional broadband UV filter. Typical TEM images of both samples are shown in Figure 1. By definition, the defect rich ZnO nanocrystals disclosed in the present invention are those ZnO nanocrystals having the ratio of the intensity λₘₐₓ of defect emission (I_{DE}) to λₘₐₓ of UV emission (I_{BEE}) above 1, i.e. I_{DE}/I_{BEE} > 1. Avobenzone-defect rich ZnO nanocrystals complex (Av-DZnONCs), where in the defect rich ZnO nanocrystal used in the present disclosure is a defect rich ZnO quantum dots (D-QDs).

The preference of ZnO nanocrystals owes it to its prominent absorbance for UV radiation in the entire spectrum of UVB and UVA radiation. However, the major problem with this ZnO material is the appearance of strong band edge emission in the UVA region which is highly undesirable for a inorganic sunscreen resulting in adverse skin problems such as erythema, hyperpigmentation, elastosis, pimples, blisters, epidermal rupture, photo- carcinogenesis and photo-ageing.

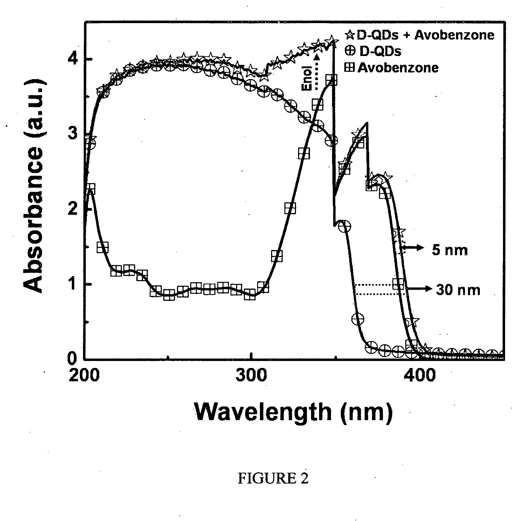

Figure 2 is representative of the Absorption spectrum of Avobenzone, D-QDs and defect rich ZnO quantum dots-avobenzone complex in accordance with an aspect of the present invention. The absorption spectrum is conclusive of the fact that the combination of D-QDs and avobenzone as disclosed in the present invention may result in an enhancement of the enol mediated absorption of avobenzone along with a red shift (∼5 nm) in the avobenzone absorption. The enhancement of UVA1 absorption and red shift in the absorption of defect rich ZnO quantum dots-avobenzone complex (D-QDs + Avobenzone) are indicative of an interaction of D-QDs with avobenzone. The defect rich ZnO nanocrystals used in the complex maybe in the size range of 1-100 nm.

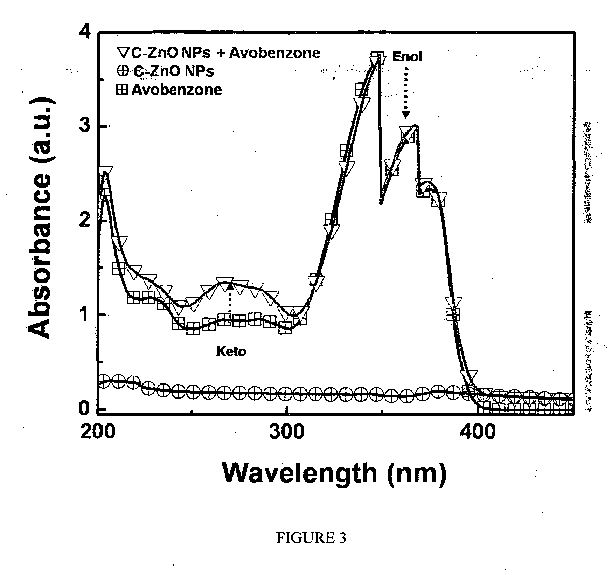

Reiterating the above fact in lieu of Avobenzone with C-ZnO NPs, Figure 3 is representative of Absorption spectrum of Avobenzone with C-ZnO NPs. Avobenzone and C-ZnONPs conclusively show enhanced absorption in the UVC range and is a deterrent. The absorption in the UVC range is due to the promotion of enol-to-keto transformation of avobenzone in the presence of C-ZnO NPs.

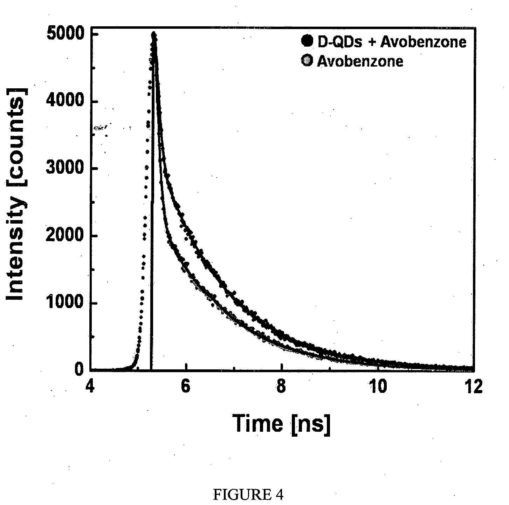

Figure 4 illustrates a decay curve for avobenzone emission. The decay curve may be obtained for studying the interaction of D-QDs with avobenzone by using time-correlated single photon counting (TCSPC). Further; the decay curve for avobenzone emission at 419 nm shows a fast decay component of 0.610 ns and in the presence of D-QDs the life time of the fast decay component increases to 0.742 ns. The increase in the fast decay component confirms a possible energy transfer in Av-DZnONCs, which possible if Av-DZnONCs nanocomplexes are formed. The TCSPC may be performed with a 395 nm laser excitation and biexponential fits to the TCSPC data (solid lines) are show in Figure 4, accordance with an aspect of the present invention.

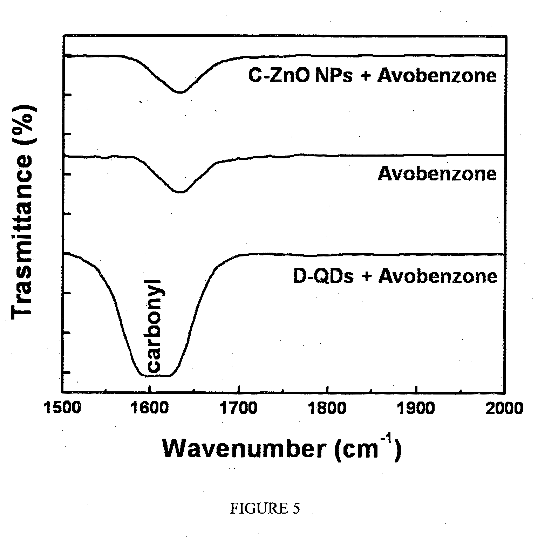

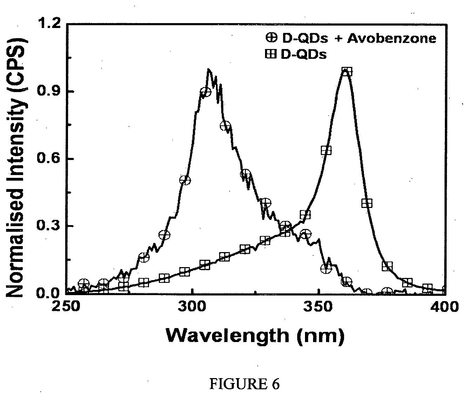

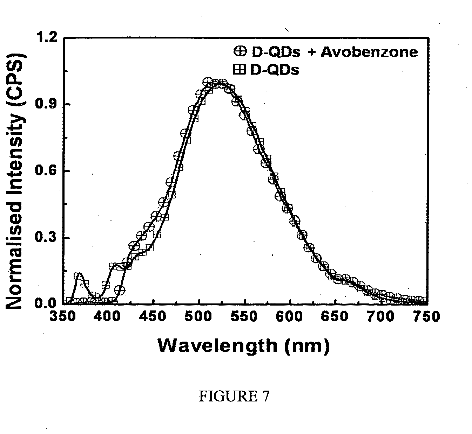

The chemical state of Av-DZnONCs complex may further be evaluated using Fourier transform infrared spectroscopy (FTIR), which shows that the formation of Av-DZnONCs complex results in an enhanced enol formation (broad peak ∼1618.38 cm⁻¹ from carbonyl absorption) when compared with the bare avobenzone and avobenzone with C-ZnO NPs as illustrated in Figure 5. Furthermore, it is observed that the excitation spectrum of Av-DZnONCs complex gets modified when compared with the D-QDs as illustrated in Figure 6, wherein excitation spectra of Av-DZnONCs and D-QDs is collected by fixing defect emission at 514 nm. Similarly a modification in the emission spectrum was observed where the weak UVA emission of D-QDs gets eliminated in the Av-DZnONCs complex as shown in Figure 7. In Figure 7, emission spectra of Av-DZnONCs complex and D-QDs collected under 350 nm excitation This elimination of weak UVA emission of D-QDs further improves the photo protective nature of Av-DZnONCs complex.

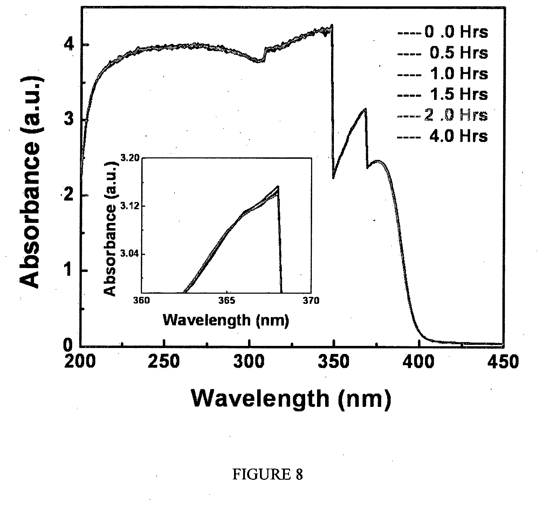

Figure 8 is representative of the absorbance spectrum showing the stability of Av-DZnONCs under 365 nm 4W UV lamp excitation monitored at different time period in accordance with an aspect of the present invention. The time dependent degradation study of Av-DZnONCs shows that Av-DZnONCs are stable even after 4 hours of UV excitation.

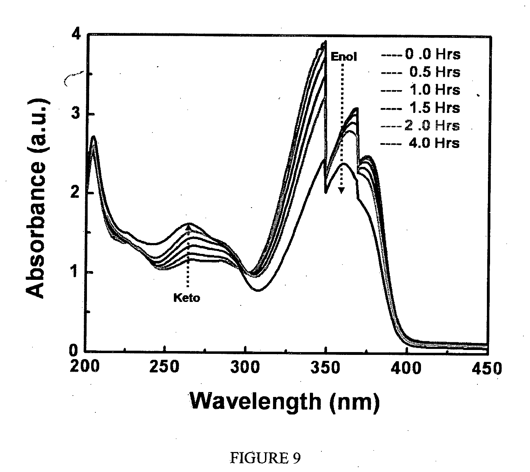

A control study with crystalline ZnO NPs (C-ZnO NPs) as shown in Figure 9 is indicative of the fact that with time the enol form is converted into keto form under UV excitation, thereby deteriorating the UVA1 absorption associated to enol form of avobenzone.

Hence, the above results justify the usage of Av-DZnONCs complex can act as an excellent photostable sunscreen active ingredient for broad spectrum UV screening.

In another aspect of this invention the cosmetic value of Av-DZnONCs complex is arrested. Ideally, inorganic sunscreen agents block UV rays by both absorbing or scattering the UV radiation. As the size of the inorganic UV filter increases the sun cream appears white in colour when applied to the skin surface. The appearance of white colour is by virtue of the increased scattering of visible light by the large sized particle used as a physical blocker. This is not desirable for the aesthetic appearance of skin surface, thereby reducing its demand in cosmetic market. Several patents have ventured into claiming the use of metal oxide nanoparticles (NPs) as an efficient UV filter, which can reduce the scattering problem faced by large sized inorganic UV filter along with an improved absorbance in the UV range. Among these metal oxide NPs most prominent sunscreen materials studied are TiO₂ and ZnO. Out of the two, the preference of ZnO nanocrystals owes it to its prominent absorbance for UV radiation in the entire spectrum of UVB and UVA radiation. The transparent ZnO NPs used as an inorganic UV filter in sunscreen has a prominent band edge emission in the UVA region (∼385 nm). The UVA emission from ZnO along with UVA radiation from sunlight can augment the UVA induced radiation effects on skin surface. Therefore, a solution to reduce the harmful UVA emission is required.

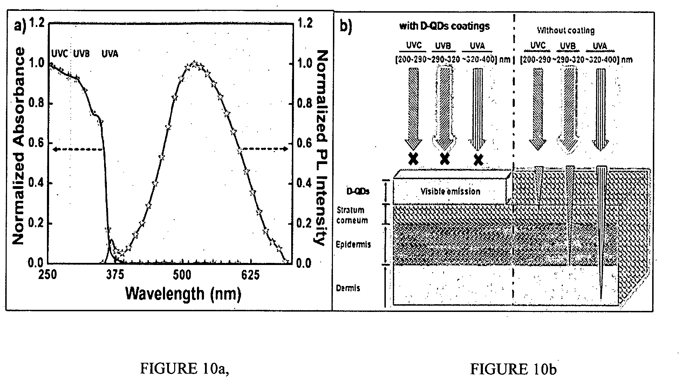

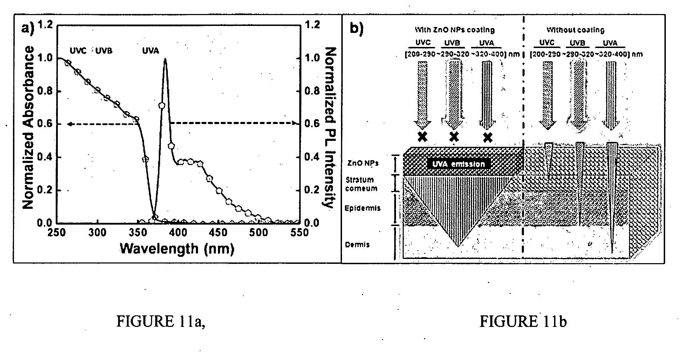

The D-QDs make use of defect energy levels as a most preferable de-excitation channel for photo-generated charge carriers produced during UV absorption, resulting in a broad and intense defect emission in the visible range as represented in Figure 10 at the expense of UVA band edge emission again depicted in Figure 11, otherwise seen in defect free/materials with fewer defects. Further, the weak UVA band edge emission of D-QDs is eliminated in combination with avobenzone. The Av-DZnONCs have no UVA emission and at the same time it possesses visible emission which could add to its cosmetic value.

The use of defect emission from D-QDs and Av-DZnONCs complex gives scope for camouflaging application in cosmetic industry. In general a normal human skin exhibits a substantial level of auto fluorescence. The auto fluorescence is found throughout the different skin layers, with the epidermis showing the weakest levels, the stratum corneum being slightly stronger, and the most intense emissions occur in dermis and subcutaneous fat (Zeng, et al., Photochem. Photobiol. 61: 639-645, 1995). The luminescence spectra of human skin measurable over a wide excitation wavelength exhibit a predominant emission in green region, as a result of strong auto fluorescence coming from elastin and collagen present in the dermis. Studies have proved that with advancing age as well as UV exposure results in reduction of these elements responsible for green luminescence.

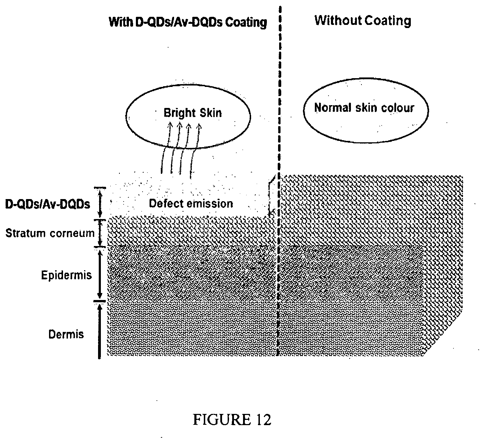

Figure 12 illustrates the use of defect emission from D-ZnO QDs for camouflaging the normal skin colour in accordance with an aspect of the present invention.

Yet again the problems associated with UV exposure and age restoration can be solved by using a multifunctional physical agent that can block UV radiation and at the same time provide visible luminescence for brightening the skin for youthful appearance. Numerous patent publication claiming the use of luminescent nanoformulations for improving the skin complexion, exist. Although these patent publications have one or more disadvantages namely having toxic heavy metal based composition, complex core shell structures, or weak emission for getting desirable brightening effect. The use of defect luminescence for cosmetic applications is rarely touched, despite of its less intrinsic toxicity as it is free from toxic transition metal or rare earth luminescent centers.

In this invention the potential of D-QDs/Av-DZnONCs complex to mimic the fluorescent effects of collagen that are lost during excess UV exposure, therefore restoring the skin's youthful appearance and offers UV protection, has been demonstrated. The D-QDs/Av-DZnONCs complex can be applied as a transparent layer in combination with an oil/water base which emits visible light giving a bright youthful skin by making wrinkles to appear shallower and masking crevices again as depicted in Figure 12.

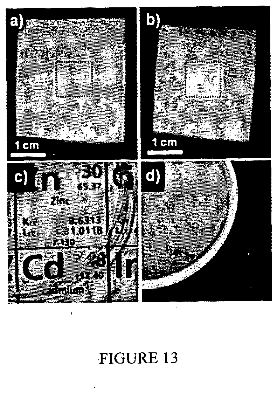

The cosmetic potential of D-QDs was explored by coating D-QDs on the surface of a skin emission model which could mimic the broad green luminescence of *in vivo* human skin. Figure 13a and 13b shows the photograph (taken under UV lamp excitation) of D-QDs (0.1 mg/cm²) and C-ZnO NPs (0.1 mg/cm²) paste (formulated in Pluronic® P-123), coated on the surface of a skin emission model. It is to be noted that the D-QDs showed a bright emission in the yellowish-green region, while the C-ZnO NPs emits in the bluish-violet range. In addition to this, an excellent transparency was observed for oleic acid capped D-QDs coated on a glass substrate (Figure 13c) under room light. This is an important sensory attribute required for a UV filter to formulate aesthetically pleasing sunscreens. Under UV excitation this transparent coating of D-QDs/Av-DZnONCs complex also showed a bright yellowish-green luminescence (Figure 13d). This bright luminescence in the yellowish-green region matches well with the *in vivo* skin autofluorescence, which further establish the cosmetic value of D-ZnO QDs. Thus the sunscreen composition containing D-QDs/Av-DZnONCs complex can be used as a safe and unique active ingredient that offers both UV protection and defect emission for camouflaging.

However, the benefits from such products depend on the appropriate use of correct amount of protective layer that is the topical composition encompassing the active ingredient. For example, sunscreens usage will help to prevent both chronic and acute damage of skin caused by solar UV radiation. In order to get efficient protection, the user should apply the correct amount of sunscreen. Studies have shown that sunscreen users fail to use the appropriate amount of sunscreen, and thus limit the benefit of its use. A report by Stokes, et al., (J. Photochemistry and Photobiology Biology, 50:137-143(1999)) introduces the use of luminescence spectroscopy for studying sunscreen performance, where the auto fluorescence from active ingredient to evaluate sunscreen performance has been used. In such a system an active ingredient responsible for absorbing ultraviolet radiation should radiatively emit in lower wavelength. Thus it allows the direct correlation of emission intensity to the concentration of active ingredient per unit area. This method can be used to predict the sun protection factor of applied sunscreen.

A similar approach was discussed in European patent application EP 2 275 176 A1; wherein the use of an additional fluorescent chromophore for measuring the sun protection factor by fluorescence spectroscopy has been highlighted. Yet again the major limitation of this method is it does not give a direct measure of active sunscreen ingredient.

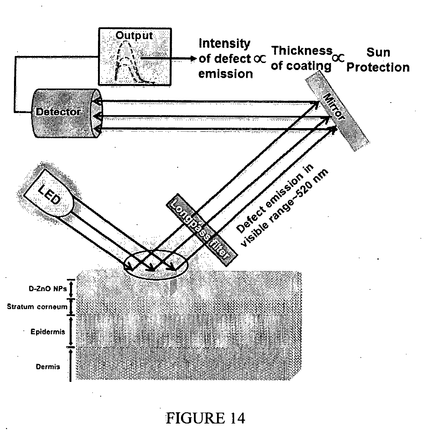

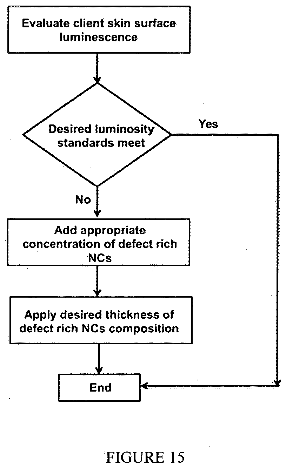

In this invention Figure 14 discloses the use of strong defect emission from D-QDs/Av-DZnONCs complex to precisely determine the sun protection factor of a surface using luminescence based detection. The other advantage of the technique is to make use of defect luminescence from D-QDs/Av-DZnONCs complex to obtain personalized cosmetic composition as per the user's skin texture. The presence of defect luminescence offers the flexibility in optimizing the desired concentration of D-QDs/Av-DZnONCs complex or thickness of D-QDs/Av-DZnONCs complex layer needed for improving skin complexion and at the same time offers good sun protection factor. A schematic description of the method of using defect rich NCs (D-QDs/Av-DZnONCs) for personalized camouflaging application in accordance with an aspect of the present invention has been described in Figure 15.

The method includes numerous steps. Initially, the luminescence of the skin surface in contention is evaluated. In the ensuing step, the skin surface is checked for meeting the luminosity standards. The luminosity standards are fixed as per the user's preference for skin texture. In an event that the desired luminosity has not been achieved; an appropriate concentration of defect rich nanocrystals is added. The concluding step is applying a desired thickness of composition comprising the defect rich nanocrystals. The above method is possible by virtue of the strong defect emission from D-QDs/Av-DZnONCs complex to precisely determine the sun protection factor of a surface using luminescence based detection.

Therefore, from the above results it can be inferred that, the Av-DZnONCs in properly formulated sunscreen composition can form a transparent and photostable layer on the skin, and under solar UV radiation it emits in the visible range giving a bright youthful appearance to skin. This visible defect luminescence of Av-DZnONCs can also find utility for camouflaging skin defects. Hence, the sunscreen composition containing Av-DZnONCs is beneficial for certain age groups and for age conscious men and women who wish to use a multifunctional cosmetic product that can provide safer sun protection and can also restore skin's youthful look.

The D-QDs described in the present invention can be prepared by any one of the known synthesis method reported in literature; by inducing inert synthesis environment, physical methods, or rapid hydrolyzes of Zn salt by creating a nonequilibrium condition or by using strong hydrolyzing agents. The Av-DZnONCs described in the present invention can be prepared by mixing desired weight% of avobenzone into a stable solution of D-QDs of known concentration. A sunscreen formulation of desired SPF and defect luminescence can be obtained by adjusting the weight% of avobenzone and D-QDs during nanocomplex formation. The composition claimed in the present invention may have a SPF value greater than 50, wherein said active ingredient (Av-DZnONCs) may be present in an amount of about 0.1 wt. % to about 30 wt. %, based on a total weight of the composition.

## Claims

1. A topical UV filter composition comprising an active ingredient of Avobenzone-defect rich ZnO nanocrystals complex (Av-DZnONCs), wherein avobenzone and defect rich ZnO nanocrystals interact to form a complex to stabilize avobenzone against photodegradation.

2. The composition as claimed in claim 1, wherein the active ingredient is from 0.1 weight percentage to 30 weight percentage of total weight of composition.

3. The composition as claimed in claim 1, wherein the active ingredient of Avobenzone-defect rich ZnO nanocrystals complex (Av-DZnONCs) is present in the form of dermatologically acceptable aqueous/oil medium.

4. The composition as claimed in claim 1, wherein the size of the defect rich ZnO nanocrystals ranges from 1 nm to 100 nm.

5. The composition as claimed in claim 1, wherein the defect rich ZnO nanocrystals is an inorganic compound.

6. The composition as claimed in claim 1, wherein the defect rich ZnO nanocrystals can be substituted by a combination of defect rich ZnO nanocrystals and another inorganic UV filter selected from a group of oxides of Zn, Zr, Al, Si, Ti, Y, Ce, Cd, Gd, Sn, Mn, Mg, In, Cu and Ga.

7. The composition as claimed in claim 1, wherein avobenzone is an organic UV filter.

8. The composition as claimed in claim 1, wherein the organic UV filter undergoes keto-to-enol transformation.

9. The composition as claimed in claim 1, wherein the defect rich ZnO nanocrystals are obtained by inducing inert synthesis environment.

10. The composition as claimed in claim 1, wherein the defect rich ZnO nanocrystals are obtained by rapid hydrolysis of Zinc salt by creating a nonequilibrium condition in the presence of strong hydrolyzing agents.

11. The composition as claimed in claim 1, wherein the Avobenzone-defect rich ZnO nanocrystals complex (Av-DZnONCs) is obtained by hydrolyzing Zn in the Avobenzone-defect rich ZnO nanocrystals complex (Av-DZnONCs).

12. The composition as claimed in claim 1, wherein the Avobenzone-defect rich ZnO nanocrystals complex (Av-DZnONCs) are stabilized even after a time duration of four hours of UV excitation.

13. The composition as claimed in claim 1, wherein an effective amount of the composition is topically applicable on a biological surface.

14. The composition as claimed in claim 1, wherein the active ingredient is present in an effective amount to improve aesthetic appearance of the biological surface, where the effective amount is in the range from 0.001 mg/cm² to 5 mg/cm²of the active ingredient.

15. The composition as claimed in claim 1, wherein ZnO nanocrystals have a ratio of the intensity λₘₐₓ of defect emission (I_{DE}) to λₘₐₓof UV emission (I_{BEE}) greater than 1.

16. A method of obtaining a personalized biological surface protection composition as per a user, the method comprising:
determining a parameter namely sun protection factor of the user's biological surface by virtue of defect emission from an Av-DZnONCs complex; evaluating luminescence of the biological surface;
ascertaining if the luminescence surpasses a pre-determined luminescence standard;
adding an optimised concentration of defect rich nanocrystals to achieve the pre-determined luminescence in the event of pre-determined luminescence standard not surpassed; wherein the optimised concentration is determined based on preference of the user for texture of the biological surface; and
providing a desired amount of personalized biological surface protection composition comprising the defect rich nanocrystals as per the user that is suitable to be applied accordingly.

17. The method of claim 16, where the avobenzone-defect rich ZnO nanocrystals complex (Av-DZnONCs) is defined according to any one of claim 1 to 15.

## Patentansprüche

1. Topische UV-Filterzusammensetzung, umfassend einen Wirkstoff von Avobenzon-defektreiche ZnO-Nanokristalle-Komplex (Av-DZnONCs), wobei Avobenzon und defektreiche ZnO-Nanokristalle interagieren, um einen Komplex zu bilden, um Avobenzon gegen Photodegradation zu stabilisieren.

2. Zusammensetzung nach Anspruch 1, wobei der Wirkstoff 0,1 Gewichtsprozent bis 30 Gewichtsprozent vom Gesamtgewicht der Zusammensetzung ausmacht.

3. Zusammensetzung nach Anspruch 1, wobei der Wirkstoff von Avobenzon-defektreiche ZnO-Nanokristalle-Komplex (Av-DZnONCs) in der Form dermatologisch akzeptablen wässrigen/öligen Mediums vorhanden ist.

4. Zusammensetzung nach Anspruch 1, wobei die Größe der defektreichen ZnO-Nanokristalle von 1 nm bis 100 nm reicht.

5. Zusammensetzung nach Anspruch 1, wobei die defektreichen ZnO-Nanokristalle eine anorganische Verbindung sind.

6. Zusammensetzung nach Anspruch 1, wobei die defektreichen ZnO-Nanokristalle durch eine Kombination aus defektreichen ZnO-Nanokristallen und einem anderen anorganischen Filter, ausgewählt aus einer Gruppe von Oxiden von Zn, Zr, Al, Si, Ti, Y Ce, Cd, Gd, Sn, Mn, Mg, In, Cu und Ga ersetzt werden können.

7. Zusammensetzung nach Anspruch 1, wobei Avobenzon ein organisches UV-Filter ist.

8. Zusammensetzung nach Anspruch 1, wobei das organische UV-Filter Keto-zu-Enol-Transformation unterliegt.

9. Zusammensetzung nach Anspruch 1, wobei die defektreichen ZnO-Nanokristalle erhalten werden, indem eine reaktionsträge Syntheseumgebung induziert wird.

10. Zusammensetzung nach Anspruch 1, wobei die defektreichen ZnO-Nanokristalle durch rasche Hydrolyse von Zinksalz erhalten werden, indem ein Ungleichgewichtszustand in der Gegenwart starker hydrolysierender Mittel geschaffen wird.

11. Zusammensetzung nach Anspruch 1, wobei der Avobenzon-defektreiche ZnO-Nanokristalle-Komplex (Av-DZnONCs) erhalten wird, indem Zn in dem Avobenzon-defektreiche ZnO-Nanokristalle-Komplex (Av-DZnONCs) hydrolysiert wird.

12. Zusammensetzung nach Anspruch 1, wobei der Avobenzon-defektreiche ZnO-Nanokristalle-Komplex (Av-DZnONCs) selbst nach einer Zeitdauer von vier Stunden an UV-Anregung stabilisiert ist.

13. Zusammensetzung nach Anspruch 1, wobei eine wirksame Menge der Zusammensetzung topisch an einer biologischen Oberfläche anwendbar ist.

14. Zusammensetzung nach Anspruch 1, wobei der Wirkstoff in einer wirksamen Menge vorhanden ist, um eine ästhetische Erscheinung der biologischen Oberfläche zu verbessern, wobei die wirksame Menge in der Spanne von 0,001 mg/cm² bis 5 mg/cm² des Wirkstoffs ist.

15. Zusammensetzung nach Anspruch 1, wobei ZnO-Nanokristalle ein Verhältnis der Intensität λₘₐₓ an Defektemission (I_{DE}) zu λₘₐₓ an UV-Emission (I_{BEE}) größer 1 aufweisen.

16. Verfahren zum Erhalten einer personalisierten biologischen Oberflächenschutzzusammensetzung für einen Anwender, wobei das Verfahren Folgendes umfasst:
Ermitteln eines Parameters, nämlich Sonnenschutzfaktors, der biologischen Oberfläche des Anwenders, mittels Defektemission von einem Av-DZnONCs Komplex; Evaluieren von Lumineszenz der biologischen Oberfläche;
Vergewissern, ob die Lumineszenz einen vorgegebenen Lumineszenzstandard übersteigt;
Hinzufügen einer optimierten Konzentration an defektreichen Nanokristallen, um die vorgegebene Lumineszenz in dem Fall zu erzielen, dass ein vorgegebener Lumineszenzstandard nicht überstiegen wird; wobei die optimierte Konzentration basierend auf Präferenz des Anwenders zur Textur der biologischen Oberfläche ermittelt wird; und
Bereitstellen einer gewünschten Menge personalisierter biologischer Oberflächenschutzzusammensetzung, umfassend die defektreichen Nanokristalle pro Anwender, die geeignet ist, entsprechend angewendet zu werden.

17. Verfahren nach Anspruch 16, wobei der Avobenzon-defektreiche ZnO-Nanokristalle-Komplex (Av-DZnONCs) nach einem der Ansprüche 1 bis 15 definiert ist.

## Revendications

1. Une composition de filtre UV local comprenant un ingrédient actif de complexe avobenzone-nanocristaux de ZnO riches en défauts (Av-DZnONCs), dans laquelle l'avobenzone et les nanocristaux de ZnO riches en défauts interagissent pour former un complexe pour stabiliser l'avobenzone vis-à-vis de la photodégradation.

2. La composition selon la revendication 1, dans laquelle l'ingrédient actif représente de 0,1 pourcent en poids à 30 pourcents en poids du poids total de la composition.

3. La composition selon la revendication 1, dans laquelle le principe actif du complexe avobenzone-nanocristaux de ZnO riche en défauts (Av-DZnONCs) est présent sous forme de milieu aqueux/huileux dermatologiquement acceptable.

4. La composition selon la revendication 1, dans laquelle la taille des nanocristaux de ZnO riches en défauts va de 1 nm à 100 nm.

5. La composition selon la revendication 1, dans laquelle les nanocristaux de ZnO riches en défauts sont un composé inorganique.

6. La composition selon la revendication 1, dans laquelle les nanocristaux de ZnO riches en défauts peuvent être remplacés par une combinaison de nanocristaux de ZnO riches en défauts et d'un autre filtre UV inorganique choisi dans un groupe d'oxydes de Zn, Zr, Al, Si, Ti, Y, Ce, Cd, Gd, Sn, Mn, Mg, In, Cu et Ga.

7. La composition selon la revendication 1, dans laquelle l'avobenzone est un filtre UV organique.

8. La composition selon la revendication 1, dans laquelle le filtre UV organique subit une transformation de céto en énol.

9. La composition selon la revendication 1, dans laquelle les nanocristaux de ZnO riches en défauts sont obtenus en induisant un environnement de synthèse inerte.

10. La composition selon la revendication 1, dans laquelle les nanocristaux de ZnO riches en défauts sont obtenus par hydrolyse rapide de sel de zinc en créant une condition de non-équilibre en présence d'agents hydrolysants puissants.

11. La composition selon la revendication 1, dans laquelle le complexe avobenzone-nanocristaux de ZnO riches en défauts (Av-DZnONCs) est obtenu par hydrolyse de Zn dans le complexe avobenzone-nanocristaux de ZnO riches en défauts (Av-DZnONCs).

12. La composition selon la revendication 1, dans laquelle le complexe avobenzone-nanocristaux de ZnO riches en défauts (Av-DZnONCs) est stabilisé même après une durée de quatre heures d'excitation UV.

13. La composition selon la revendication 1, dans laquelle une quantité efficace de la composition est applicable localement sur une surface biologique.

14. La composition selon la revendication 1, dans laquelle l'ingrédient actif est présent en une quantité efficace pour améliorer l'aspect esthétique de la surface biologique, dans laquelle la quantité efficace est située dans la gamme allant de 0,001 mg/cm² à 5 mg/cm² de l'ingrédient actif.

15. La composition selon la revendication 1, dans laquelle les nanocristaux de ZnO ont un rapport de l'intensité λₘₐₓ d'émission de défaut (I_{DE}) à λₘₐₓ d'émission UV (I_{BEE}) supérieur à 1.

16. Procédé d'obtention d'une composition de protection de surface biologique personnalisée selon un utilisateur, le procédé comprenant les étapes de:
déterminer un paramètre, à savoir un facteur de protection solaire de la surface biologique de l'utilisateur grâce à l'émission de défauts d'un complexe Av-DZnONCs ; évaluer la luminescence de la surface biologique ;
vérifier si la luminescence dépasse un standard de luminescence prédéterminé ;
ajouter une concentration optimisée de nanocristaux riches en défauts pour obtenir la luminescence prédéterminée dans le cas où le standard de luminescence prédéterminée n'est pas dépassé ; dans lequel la concentration optimisée est déterminée sur la base d'une préférence de l'utilisateur pour la texture de la surface biologique ; et
fournir une quantité souhaitée de composition de protection de surface biologique personnalisée comprenant les nanocristaux riches en défauts en fonction de l'utilisateur, qu'il est approprié d'appliquer en conséquence.

17. Le procédé selon la revendication 16, dans lequel le complexe avobenzone-nanocristaux de ZnO riches en défauts (Av-DZnONCs) est défini selon l'une quelconque des revendications 1 à 15.
